(19) 

Europäisches
Patentamt
European
Patent Office
Office européen
des brevets

(11)  **EP 4 610 352 A1**

(12)  **EUROPEAN PATENT APPLICATION**
published in accordance with Art. 153(4) EPC

(43) Date of publication:
**03.09.2025  Bulletin 2025/36**

(21) Application number: **23885011.9**

(22) Date of filing: **01.11.2023**

(51) International Patent Classification (IPC):
**C12N 9/52** *(2006.01)*     **A61K 38/48** *(2006.01)*
**A61P 35/00** *(2006.01)*    **A61P 11/06** *(2006.01)*

(52) Cooperative Patent Classification (CPC):
**A61K 38/48; A61K 38/00; A61K 45/06;
A61P 11/06; A61P 35/00; C12N 9/52;** C12Y 304/21

(86) International application number:
**PCT/CN2023/129169**

(87) International publication number:
**WO 2024/094085 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **01.11.2022  CN 202211358492**

(71) Applicant: **Sun, Zhenglong
Suzhou, Jiangsu 215004 (CN)**

(72) Inventors:
• **SUN, Zhenglong
   Suzhou, Jiangsu 215004 (CN)**

• **LIU, Jun
   Suzhou, Jiangsu 215004 (CN)**
• **ZHANG, Cong
   Suzhou, Jiangsu 215004 (CN)**
• **CHEN, Kai
   Suzhou, Jiangsu 215004 (CN)**

(74) Representative: **Dr. Gassner & Partner mbB
Wetterkreuz 3
91058 Erlangen (DE)**

Remarks:
The complete document including Reference
Table(s) and the Sequence Listing(s) can be
downloaded from the EPO website

(54)  **SERINE PROTEASE FOR SELECTIVELY DEGRADING MUCIN AND USE THEREOF**

(57)  The present application relates to a serine protease for selectively degrading mucin and use thereof. The serine protease is serine protease EatA, and the protein thereof comprises a passenger domain with serine protease EatA activity. The passenger domain has an amino acid sequence with at least 90% identity with SEQ ID NO. 2, and is preferably an amino acid sequence set forth in SEQ ID NO. 2. The serine protease EatA can selectively degrade mucin in disease-related mucus, and has relatively high and lasting stability.

FIG. 3

EP 4 610 352 A1

**Description**

**TECHNICAL FIELD**

The present application relates to a technical field of biomedicine, in particular to a serine protease selectively degrading mucins and use thereof in mucin degradation.

**BACKGROUND ART**

Mucins are a class of high molecular weight and highly glycosylated proteins expressed in humans by secretory cells typically found in epithelial glandular tissue of gastrointestinal tract, respiratory tract, lung, kidney, ovary, breast, and pancreas. Mucins act in normal physiological environments to lubricate and protect human tissue surfaces. The expression and composition of mucins are tightly regulated. However, under some pathological conditions, such as cancer, and inflammation, the expression and composition of mucins are altered without control, and the large accumulation of these mucins has a great negative impact on patients. Severe clinicopathologic features are present in both cancer and non-cancerous diseases due to aberrant expression of mucins. The above-mentioned diseases include peritoneal pseudomyxoma, chronic obstructive pulmonary disease, mucinous colorectal cancer, mucinous ovarian cancer, etc.

Peritoneal pseudomyxoma is a kind of cancer with unknown etiology, but the clinical diagnosis mostly originates from organs and tissues such as ovary, appendix, and intestinal mucous gland. Cancer cells eventually colonize the peritoneal surface of peritoneum and abdominal organs. Tumors continue to grow and secrete large amounts of mucin, especially mucin MUC2. MUC2 is a major gel-forming mucin in the small and large intestine. Gel-forming mucins form gel-like jelly-like polymers at specific pH values, salt concentrations, and compositions of homologous mucin concentrations. The core region of MUC2 molecule is highly glycosylated and highly resistant to degradation by conventional proteases such as proteinase K, and gastric trypsin, and covalent interactions at the C-terminal and N-terminal of the protein chain facilitate the formation of MUC2 dimers and multimers, respectively. Gel-like mucins encapsulate lesions such as cancer cell mass and gradually develop into diffuse metastases, forming peritoneal pseudomyxoma, mucus is widespread within the peritoneum. Since this type of cancer cell is encapsulated by a large amount of gel-like mucins, the conventional drug delivery mode makes it difficult for the drug to reach the target, and the therapeutic effect of the drug is poor. The large amount of mucin in the abdominal cavity distributed in patient's body causes the patient to suffer, in severe cases, a large amount of mucin compresses organs, leading to intestinal obstruction complications and endangering patients' lives.

Chronic obstructive pulmonary disease (COPD) is a chronic pulmonary disease and the third leading cause of death in the world (2019, WTO). Abnormal small airways in the lungs result in restricted airflow into and out of lungs. Causes of chronic obstructive pulmonary disease include chronic exposure to harmful gases and particulates, as well as personal factors such as events affecting lung development during childhood and genetic factors. Chronic obstructive pulmonary disease causes persistent and progressive respiratory symptoms including dyspnea, cough, and sputum production. Some processes lead to airway narrowing. Part of the lungs may be damaged, and patients with this disease have abnormal mucus secretion in the respiratory tract, which can block the airway and cause inflammation and swelling of a wall of the airway. Chronic obstructive pulmonary disease is also sometimes referred to as "emphysema" or "chronic bronchitis". Emphysema usually refers to damage to the small alveoli at the end of the airway in the lungs. Chronic bronchitis refers to a chronic cough caused by inflammation of the airway, accompanied by sputum production.

The etiology of mucinous ovarian cancer is not clear. The origin of mucinous ovarian cancer is mostly from mucus epithelial cells, which leads to bilateral ovarian involvement, ovarian surface involvement, mucins extracellularly, destructive interstitial infiltration, nodular growth, ovarian hilum involvement, vascular invasion, signet ring cells, and extensive necrosis in patients. More than 90% of ovarian mucinous tumors contain a large amount of mucin in cells.

In addition, mucinous colorectal cancer and other viscous diseases, all have pathological properties accompanied by massive expression of mucus. There is therefore a need to remove mucins in the treatment of diseases involving mucins in order to provide patients with better results. For example, in the treatment of peritoneal pseudomyxoma, mucus can be removed through surgical laparotomy, but is often difficult to remove cleanly, and long-term, multiple minimal invasive open operations are a burden on the patient, thus mucolytic reagents have been developed and used to treat mucin-related diseases. The above-mentioned reagents can break down the structure of mucins by physical and chemical means, remove the gel-like structure, so that mucins can be easily sucked out of the body, or removed by the human body, or drugs promoting cancer cell targeting can reach the target cells more easily to perform the anti-cancer effect. Active patients who are clinically ready for surgery have extensive intra-abdominal tumors and hard mucus, leading to severe organ adhesion, increasing the difficulty of surgery and the incidence of adverse events. Therefore, softening or even dissolving the mucus before surgery can reduce the degree of organ adhesion to some extent and reduce the risk of surgery.

Thus, it can be seen that mucolytic reagents have an important role in the treatment of mucin-related diseases, and there is a need to provide new proteases and preparations thereof that better degrade mucins associated with diseases.

## SUMMARY

In order to solve deficiencies in the prior art, the present application provides a serine protease EatA for selectively degrading mucins based on a discovery of the degrading activity of the serine protease EatA, wherein the serine protease EatA can effectively degrade mucins in mucus-related diseases, and can exert a mucus degrading function of the serine protease EatA after being administered to a patient, and thus can be preferably used in the treatment of mucus-related diseases.

To this end, in a first aspect, the present application provides a serine protease selectively degrading mucins, wherein the serine protease is a serine protease EatA, and a protein of the serine protease EatA includes a passenger domain with serine protease EatA activity, and an amino acid sequence of the passenger domain is an amino acid sequence with at least 90% identity to SEQ ID NO 2.

In some specific embodiments, the amino acid sequence of the passenger domain is an amino acid sequence with 90%, 91%, 92%, 93%, 94%, 95%, 96%, 97%, 99%, or 100% identity to SEQ ID NO 2.

In some preferred embodiments, the amino acid sequence of the passenger domain is an amino acid sequence as shown in SEQ ID NO 2. The specific sequence of SEQ ID NO 2 is as follows:

ATVNADISYQTFRDFAENKGAFIVGASNINIYDKNGVLVGVLDKAPMPDFSSAT

MNTGTLPPGDHTLYSPQYVVTAKHVNGSDIMSFGHIQNNYTVVGENNHNSLDIKIRRLN

KIVTEVAPAEISSVGAVNGAYQEGGRFKAFYRLGGGLQYIKDKNGNLTPVYTNGGFLTG

GTISALSSYNNGQMITAPTGDIFNPANGPLANYLNKGDSGSPLFAYDSLDKKWVLVGVLS

SGSEHGNNWVVTTQDFLHQQPKHDFDKTISYDSEKGSLQWRYNKNSGVGTLSQESVV

WDMHGKKGGDLNAGKNLQFTGNNGEIILHDSIDQGAGYLQFFDNYTVTSLTDQTWTG

GGIITEKGVNVLWQVNGVNDDNLHKVGEGTLTVNGKGVNNGGLKVGDGTVILNQRPD

DNGHKQAFSSINISSGRATVILSDANQVNPDKISWGYRGGTLDLNGNNVNFTRLQAADY

GAIVSNNNKNKSELTLKLQTLNENDISVDVKTYEVFGGHGSPGDLYYVPASNTYFILKSK

AYGPFFSDLDNTNVWQNVGHDRDKAIQIVKQQKIGESSQPYMFHGQLNGYMDVNIHPL

SGKDVLTLDGSVNLPEGVITKKSGTLIFQGHPVIHAGMTTSAGQSDWENRQFTMDKLRL

DAATFHLSRNAHMQGDISAANGSTVILGSSRVFTDKNDGTGNAVSSVEGSSIATTAGDQS

YYSGNVLLENHSSLEVRENFTGGIEAYDSSVSVTSQNAIFDHVGSFVNSSLLLEKGAKLT

AQSGIFTNNTMKIKENASLTLTGIPSVGKPGYYSPVTSTTEGIHLGERASLSVKNMGYLSS

NITAENSAAIINLGDSNATIGKTDSPLFSTLMRGYNAVLQGNIMGPQSSVNMNNALWHS

DRNSELKELKANDSQIELGVRGHFAKLRVKELIASNSVFLVHANNSQADQLNVTDKLQG

SNNTILVDFFNKAANGTNVTLITAPKGSDENTFKAGTQQIGFSNITPEIRTENTDTATQWV

LTGYQSVADARASKIATDFMDSGYKSFLTEVNNLNKRMGDLRD.

In the present application, since the passenger domain in the serine protease EatA protein is correctly folded, the activity of the serine protease EatA (i.e. the activity of selectively degrading mucins), which is formed by the histidine-aspartic acid-serine catalytic triad, is possessed. Thus, in some specific embodiments, the serine protease EatA is a serine protease EatA-EatAp with only the passenger domain.

In some embodiments, the serine protease EatA further includes at least one of a signal peptide at an N-terminal and a beta barrel structure at a C-terminal.

In some embodiments, an amino acid sequence of the serine protease EatA with the passenger domain and the beta barrel structure at the C-terminal is an amino acid sequence with at least 90% identity to SEQ ID NO 3.

In some specific embodiments, the amino acid sequence of the serine protease EatA with the passenger domain and the beta barrel structure at the C-terminal is an amino acid sequence with 90%, 91%, 92%, 93%, 94%, 95%, 96%, 97%, 99% or 100% identity to SEQ ID NO 3.

In some preferred embodiments, the amino acid sequence of the serine protease EatA with the passenger domain and the beta barrel structure at the C-terminal is an amino acid sequence as shown in SEQ ID NO. 3. The specific sequence of SEQ ID NO 3 is as follows:

ATVNADISYQTFRDFAENKGAFIVGASNINIYDKNGVLVGVLDKAPMPDFSSAT

MNTGTLPPGDHTLYSPQYVVTAKHVNGSDIMSFGHIQNNYTVVGENNHNSLDIKIRRLN

KIVTEVAPAEISSVGAVNGAYQEGGRFKAFYRLGGGLQYIKDKNGNLTPVYTNGGFLTG

GTISALSSYNNGQMITAPTGDIFNPANGPLANYLNKGDSGSPLFAYDSLDKKWVLVGVLS

SGSEHGNNWVVTTQDFLHQQPKHDFDKTISYDSEKGSLQWRYNKNSGVGTLSQESVV

WDMHGKKGGDLNAGKNLQFTGNNGEIILHDSIDQGAGYLQFFDNYTVTSLTDQTWTG

GGIITEKGVNVLWQVNGVNDDNLHKVGEGTLTVNGKGVNNGGLKVGDGTVILNQRPD

DNGHKQAFSSINISSGRATVILSDANQVNPDKISWGYRGGTLDLNGNNVNFTRLQAADY

GAIVSNNNKNKSELTLKLQTLNENDISVDVKTYEVFGGHGSPGDLYYVPASNTYFILKSK

AYGPFFSDLDNTNVWQNVGHDRDKAIQIVKQQKIGESSQPYMFHGQLNGYMDVNIHPL

SGKDVLTLDGSVNLPEGVITKKSGTLIFQGHPVIHAGMTTSAGQSDWENRQFTMDKLRL

DAATFHLSRNAHMQGDISAANGSTVILGSSRVFTDKNDGTGNAVSSVEGSSIATTAGDQS

YYSGNVLLENHSSLEVRENFTGGIEAYDSSVSVTSQNAIFDHVGSFVNSSLLLEKGAKLT

AQSGIFTNNTMKIKENASLTLTGIPSVGKPGYYSPVTSTTEGIHLGERASLSVKNMGYLSS

NITAENSAAIINLGDSNATIGKTDSPLFSTLMRGYNAVLQGNIMGPQSSVNMNNALWHS

DRNSELKELKANDSQIELGVRGHFAKLRVKELIASNSVFLVHANNSQADQLNVTDKLQG

SNNTILVDFFNKAANGTNVTLITAPKGSDENTFKAGTQQIGFSNITPEIRTENTDTATQWV

LTGYQSVADARASKIATDFMDSGYKSFLTEVNNLNKRMGDLRDSQGDAGGWARIMNG

TGSGESGYRDNYTHVQIGADRKHELNGIDLFTGALLTYTDNNASSQAFSGKTKSLGGGV

YASGLFESGAYFDLIGKYLHHDNRYTLNFASLGERSYTSHSLYAGAEIGYRYHMSENTW

VEPQMELVYGSVSGKSFNWKDQGMQLSMKDKDYHPLIGRTGVDVGRAFSGDTWKVT

VRAGLGYQFDLLANGETVLQDASGKKHFKGEKDSRMLMNVGTNVEVKDNMRFGLEL

EKSAFGRYNIDNSINANFRYYF* (terminator codon).

In some embodiments, a full-length amino acid sequence of the serine protease EatA with the passenger domain, the signal peptide at the N-terminal, and the beta barrel structure at the C-terminal is an amino acid sequence with at least 80% identity to SEQ ID NO 1.

In some specific embodiments, the full-length amino acid sequence of the serine protease EatA with the passenger domain, the signal peptide at the N-terminal and the beta barrel structure at the C-terminal is an amino acid sequence with 80%, 81%, 82%, 83%, 84%, 85%, 86%, 87%, 88%, 89%, 90%, 91%, 92%, 93%, 94%, 95%, 96%, 97%, 99% or 100% identity to SEQ ID NO 1.

In some preferred embodiments, the full-length amino acid sequence of the serine protease EatA with the passenger domain, the signal peptide at the N-terminal, and the beta barrel structure at the C-terminal is an amino acid sequence with at least 90% identity to SEQ ID NO 1.

In some more preferred embodiments, the full-length amino acid sequence of the serine protease EatA with the passenger domain, the signal peptide at the N-terminal and the beta barrel structure at the C-terminal is an amino acid sequence as shown in SEQ ID NO 1. The specific sequence of SEQ ID NO 1 is as follows:

MNKVFSLKYSFLAKGFIAVSELARRVSVKGKLKSASSIIISPITIAIVSYAPPSLAA

TVNADISYQTFRDFAENKGAFIVGASNINIYDKNGVLVGVLDKAPMPDFSSATMNTGTL

PPGDHTLYSPQYVVTAKHVNGSDIMSFGHIQNNYTVVGENNHNSLDIKIRRLNKIVTEVA

PAEISSVGAVNGAYQEGGRFKAFYRLGGGLQYIKDKNGNLTPVYTNGGFLTGGTISALSS

YNNGQMITAPTGDIFNPANGPLANYLNKGDSGSPLFAYDSLDKKWVLVGVLSSGSEHGN

NWVVTTQDFLHQQPKHDFDKTISYDSEKGSLQWRYNKNSGVGTLSQESVVWDMHGK

KGGDLNAGKNLQFTGNNGEIILHDSIDQGAGYLQFFDNYTVTSLTDQTWTGGGIITEKG

VNVLWQVNGVNDDNLHKVGEGTLTVNGKGVNNGGLKVGDGTVILNQRPDDNGHKQ

AFSSINISSGRATVILSDANQVNPDKISWGYRGGTLDLNGNNVNFTRLQAADYGAIVSN

NNKNKSELTLKLQTLNENDISVDVKTYEVFGGHGSPGDLYYVPASNTYFILKSKAYGPFF

SDLDNTNVWQNVGHDRDKAIQIVKQQKIGESSQPYMFHGQLNGYMDVNIHPLSGKDV

LTLDGSVNLPEGVITKKSGTLIFQGHPVIHAGMTTSAGQSDWENRQFTMDKLRLDAATF

HLSRNAHMQGDISAANGSTVILGSSRVFTDKNDGTGNAVSSVEGSSIATTAGDQSYYSG

NVLLENHSSLEVRENFTGGIEAYDSSVSVTSQNAIFDHVGSFVNSSLLLEKGAKLTAQSGI

FTNNTMKIKENASLTLTGIPSVGKPGYYSPVTSTTEGIHLGERASLSVKNMGYLSSNITAE

NSAAIINLGDSNATIGKTDSPLFSTLMRGYNAVLQGNIMGPQSSVNMNNALWHSDRNSE

LKELKANDSQIELGVRGHFAKLRVKELIASNSVFLVHANNSQADQLNVTDKLQGSNNTI

LVDFFNKAANGTNVTLITAPKGSDENTFKAGTQQIGFSNITPEIRTENTDTATQWVLTGY

QSVADARASKIATDFMDSGYKSFLTEVNNLNKRMGDLRDSQGDAGGWARIMNGTGSG

ESGYRDNYTHVQIGADRKHELNGIDLFTGALLTYTDNNASSQAFSGKTKSLGGGVYASG

LFESGAYFDLIGKYLHHDNRYTLNFASLGERSYTSHSLYAGAEIGYRYHMSENTWVEPQ

MELVYGSVSGKSFNWKDQGMQLSMKDKDYHPLIGRTGVDVGRAFSGDTWKVTVRAG

LGYQFDLLANGETVLQDASGKKHFKGEKDSRMLMNVGTNVEVKDNMRFGLELEKSAF

GRYNIDNSINANFRYYF* (terminator codon).

In the present application, the serine protease EatA is an autotransporter derived from enterotoxigenic Escherichia coli of Enterobacteriaceae, and a full-length protein of the serine protease EatA includes the signal peptide at the N-terminal, the beta barrel structure at the C-terminal and the passenger domain in the middle of a protein chain, wherein the passenger domain has a serine protease activity, and the activity is formed by a histidine-aspartic acid-serine catalytic triad; a serotype of Enterobacteriaceae is O78: H11, a enterotoxigenic Escherichia coli strain is H10407 strain. As used herein, the serine protease EatA is defined as: a protein including the passenger domain fragment EatA-EatAp having serine protease activity as shown in SEQ ID NO 2, the full-length protein of the serine protease EatA as shown in the sequence of SEQ ID NO 1, and a protease fragment without the signal peptide as shown in the sequence of SEQ ID NO 3.

In the present application, the serine protease EatA is expressed in Escherichia coli as the expression vector, the full length of EatA is a membrane protein, a middle part (passenger domain) is transferred to the outside of the membrane by means of self-transport, and the passenger domain is released by self-cleavage, and the protein released in the medium is the target protein of EatA with serine protease activity (serine protease EatA-EatAp with only passenger domain).

Mucolytic reagents are protease-containing reagents that affect and act on mucins by causing proteolysis of glycoproteins and mucins, to effect degradation of the gelled mucus. The serine protease EatA, a member of the serine autotransporter family of Enterobacteriaceae, has been used as an antigen to prevent severe diarrhea caused by Diarrhoeal Enterobacter and to produce antibodies by injection to elicit an immune response. The inventors of the present application have found that the serine protease EatA has a considerable stationary phase in vitro and can disrupt the gel-like physical state of mucins to form a liquid state. Thus the serine protease EatA of the present application can be used to prepare mucolytic reagents.

It should be noted that homologues of the serine protease EatA can be found in other species and species by conventional and known search methods in the art, and that sequence similarity of homologues can be determined by conventional algorithms. For example, when comparing the similarity of two proteins, it can be calculated by using the widely used blastN and blastX programs, algorithms of which are derived from Karlin and Altschul, allowing the introduction of a small number of gaps to achieve the best fit of the percent sequence identity between two nucleic acid or protein molecules. Based on the above, therefore, the serine protease EatA described in the present application can also be replaced by a homologue having a high degree of identity (at least 80% identity) to the amino acid sequence of the serine protease EatA.

In some embodiments, the optimal reaction conditions for the serine protease EatA to degrade mucins are: the reaction temperature is 34-37°C, the pH value is 7-8, and the reaction coefficient is 1 to 1,000,000. For example, in some specific embodiments, the serine protease EatA degrades mucins at a reaction temperature of 37°C and a pH value of 7.2-7.4 (a pH value of 7.2-7.4 is more consistent with the human environment, especially tissue and abdominal pH), and a reaction coefficient of 100 to 10000.

In the present application, when the reaction conditions are controlled within the above-mentioned range, the degradation effect of mucins by the serine protease EatA in an in vitro experiment is the best.

Since the mucins degraded in the present application is a gel-like semi-solid, which is initially insoluble in water, but has slow hydration, the relative average density of the mucins and water used in examples of the present application is close to 1.0, so we make the following definition when measuring the concentration. Reaction coefficient: in a fixed 1000uL (1, y) reaction system with 1000uL (1, x) of mucin, the density of mucin is 1 (1 g/mL, ρ), and the system contains a total mass concentration of 1ug/mL (1ug, z) of serine protease EatA; after incubation at 37 °C for 10 hours, a mucin dissolution value w=100% indicates that the degradation is complete (the w represents a percentage of the mass of the mucus dissolved), and the reaction coefficient is 1. Namely, the calculation formula of the reaction coefficient is:

$$\text{Equation 1: -------------------reaction coefficient } \delta = \frac{y}{x*z} * w * \rho; \; y > x, \; y \sim \delta:$$

that is to say, in the case where a ratio between the amount of enzyme and the amount of substrate mucins is known, a volume of the reaction system cannot exceed a corresponding reaction coefficient of the reaction system, and a volume value of the reaction system should always be greater than a volume value of mucin.

Therefore, it can be foreseen that a range of reaction coefficients used is 1 to 1 million, such as 1 to 100, 100 to 1000, 1000 to 10000, 10000 to 100,000, 100,000 to 1,000,000n, etc.

The construction of the above reaction coefficient is based on logic, since mucins spontaneously hydrate in a large volume of aqueous solution and dissolve slowly, in order to reduce an effect of hydration on enzymatic degradation, the reaction coefficient refers to a non-linear effect intensity of the reaction system on the substrate mucins when all the mucus can be degraded into soluble liquid 10 hours after the addition of serine protease EatA, and cannot directly represent an enzyme activity. The value of the reaction coefficient is related to the reaction volume, the amount of substrate mucin, the mass concentration and activity of the enzyme, and the reaction time. Since mucins have different densities in different pathologies, their volumes are used uniformly as a marker scale for calculations.

It should be noted that the reaction coefficients in the examples are preferred and are not intended to indicate and limit the amounts and concentrations at which EatA degrades mucus/mucins.

In some embodiments, the serine protease EatA has a specific serine protease activity for selectively degrading mucin.

In the present application, the serine protease EatA is capable of specifically degrading mucins but does not have a degrading function for non-target proteins, such as miscellaneous proteins in mucus. In previous studies, EatA did not degrade common human glycoproteins such as gelatin, lactoferrin and CD43, as well as non-human proteins such as BSA and BSM; the experimental results of the inventors of the present application further show that EatA does not degrade the miscellaneous protein contained in mucins of clinical origin, but only selectively degrades gel-like mucins, converting the gel-like mucins from a semi-solid gel-like to a solution, easily transported by sucking or possibly absorbed by the body. Therefore, the serine protease EatA in the present application can be better used to degrade mucins in mucus-related diseases without causing damage to other proteins in the body.

In some embodiments, the mucins degraded is at least one of mucins present in peritoneal pseudomyxoma mucus, chronic obstructive pulmonary disease mucus, mucinous ovarian cancer mucus, mucinous colorectal cancer mucus, and appendix cancer mucus; preferably present in peritoneal pseudomyxoma mucus.

In the present application, the mucins degraded by the serine protease EatA are not denatured purified mucins (such as MUC2), but mucins present in disease-related mucus. Unlike mucins purified by denaturation (e.g. MUC2), mucins present in disease-associated mucus participate in the formation of mucus with tissues, particularly gel-like mucus in PMP, MUC2 forms large aggregates with itself and other mucins family molecules, and mucins families have different modes of modification in disease. Thus, mucins present in disease-associated mucus are more complex in structure and more difficult to degrade than denatured purified mucins. For example, elastase and pancreatin, both degrade mucin molecules purified to SDS-PAGE grade but are inefficient for mucin degradation after mucus formation. However, the serine protease EatA described in the present application can efficiently degrade mucins in mucus-related diseases, and can further be used in mucus-related diseases such as peritoneal pseudomyxoma expressing the above-mentioned mucins, especially expressing MUC2-type mucins, so as to degrade mucins, eliminate gel properties and achieve specific therapeutic effects.

In the present application, the specific therapeutic effects include, but are not limited to, forms that facilitate mucin clearance, expose cancer cells or lesions, promote the effects of other chemotherapeutic drugs, and reduce patient involvement.

When the serine protease EatA in the present application is used for treating mucous diseases such as peritoneal pseudomyxoma, since the serine protease EatA in the present application has a long and stable activity, after being infused into the abdominal cavity of a human body, the serine protease EatA has a long-lasting mucus degradation activity, reduces the mass and volume of a gel-like solid formed by a large number of mucins by digestion under minimally invasive or even non-invasive conditions, is easier to perform and is less invasive than laparotomy, thus avoiding the possible infection and injury to the patient caused by laparotomy. Degradation of mucus by the serine protease EatA can be achieved by infusion into specific pathological areas of the patient's body in a manner that depends on the type of disease and the target area. As a specific example, in the case of the covered pseudomyxoma, the degradation of mucus can be achieved by means of injection, directly by injecting a specific concentration of EatA protease into the abdominal cavity, or into a tumor or cancerous cyst, so as to achieve degradation of mucins in the abdominal cavity or target region, and finally achieve a certain therapeutic effect. This therapeutic effect may be a decrease in the amount of mucins in the environment in which it is located and at the same time an improvement in the patient's involvement.

It should be noted that when the serine protease EatA described in the present application degrades the mucus of mucus-related diseases, the degraded mucus becomes a liquid similar to water, eliminating the gel property of mucus, so that the mucus in the lesion area of the patient can be easily removed, can be sucked out from the body through a needle or a specific suction device, and can promote other therapeutic drugs such as anti-cancer drugs to directly reach the lesion and cancer cells, thereby improving the therapeutic efficacy, reducing the involvement and burden of patients and improving the quality of life. Compared with existing preparations for degrading mucins in the art (such as preparations including bromelain), the serine protease EatA described in the present application has better properties, exhibits superior selectivity, does not degrade non-target proteins, has a higher degradation efficiency and more degradation products in line with degradation expectations, such as being more easily absorbed by pipette instruments.

A second aspect of the present application provides a pharmaceutical composition including the serine protease as described in the first aspect of the present application.

In some embodiments, the pharmaceutical composition further includes an additional reagent, and the additional reagent is at least one selected from a group consisting of: a chemotherapeutic and radiotherapeutic reagent, an enzyme, and a chemical salt reagent.

In the present application, the serine protease EatA can be used in combination with one or more of other reagents with therapeutic effects, such as common cytotoxic chemotherapeutic drugs, e.g. cisplatin and gemcitabine, so that the drug can more directly and effectively target cancer cells and improve the effectiveness of chemotherapy. In addition, the mucus-degrading function of the serine protease EatA is its protease activity that is inhibited by PMSF, and thus in specific implementations, the serine protease EatA may be used in combination with some enzymes having other mucins sugar chain degrading activity and some chemical salt reagents.

In some specific embodiments, the chemotherapeutic and radiotherapeutic reagent is at least one selected from a group consisting of: gemcitabine, cisplatin, adriamycin, fluorouracil, taxodone, paclitaxel, and oxaliplatin; the enzyme is at least one selected from a group consisting of: N-acetylgalactosidase, galactosidase, glucosidase, sialidase and specific endomucinase; and the chemical salt reagent is at least one selected from a group consisting of: sodium bicarbonate, carbocysteine, N-acetylcysteine and ambroxol.

A third aspect of the present application provides a use of the serine protease as described in the first aspect of the present application or the pharmaceutical composition as described in the second aspect of the present application in preparing a drug for treating mucus-related diseases, and/or for improving a therapeutic effect of the mucus-related diseases and a quality of life of a patient with mucus-related diseases.

Since the protease activity of the serine protease EatA, the serine protease EatA can be used as a drug or pharmaceutical ingredient for treating mucus-related diseases, the present application provides a new use of the serine protease EatA in the mucus-related diseases such as peritoneal pseudomyxoma, chronic obstructive pulmonary disease. Treatment of mucus-related diseases can be achieved by administering an effective amount of the serine protease EatA to focal areas of the above-mentioned mucus-related diseases, such as the abdominal cavity and respiratory tract, resulting in degradation of mucins in the focal areas, or reduction or depletion of the amount of gel-like proteins such that a large number of mucins can be easily removed. Further, after the mucins are degraded by administering the effective amount of the serine protease EatA, the encapsulation and isolation of cancer cells and lesions by mucus are eliminated, resulting in enhanced efficacy of cytotoxic drugs, enhanced accessibility to lesions and cancer cells by other drugs such as the chemotherapeutic drugs cisplatin, gemcitabine, and enhanced the therapeutic effect of mucus-related diseases and patient quality of life. Therefore, the serine protease described in the first aspect or the pharmaceutical composition described in the second aspect of the present application can be used in preparing drugs for treating mucus-related diseases, and/or drugs for improving the therapeutic effect of mucus-related diseases and the quality of life of patients with mucus-related diseases, and the drugs including the serine protease described in the first aspect or the pharmaceutical composition described in the second aspect can improve the therapeutic effect by degrading mucins and destroying the gel-like protein formed by mucin, making the mucins easy to be cleared, and promoting other therapeutic drugs such as anti-cancer drugs to directly reach lesions and cancer cells, thereby reducing the involvement and burden of patients and improving the quality of life.

In the present application, the above-mentioned mucin-related diseases, except for mucinous carcinoma as defined by the World Health Organization, which is a malignant tumor composed of gastrointestinal type cells containing intracytoplasmic mucin, can also be considered pathological tissues formed by abnormal regulation of mucin expression in cells, tissues, and organs under normal health conditions due to functional or environmental changes, resulting in the production of a large amount of mucus, or the secretion of mucin that wraps around the outside of cells, tissues, and organs, blocking the realization of normal physiological functions (such as immune recognition), or the infiltration and access of therapeutic drugs.

In some embodiments, the mucus-related disease is at least one selected from a group consisting of: chronic obstructive pulmonary disease, mucinous colorectal cancer, lung cancer, liver cancer, gastric cancer, appendix cancer, peritoneal cancer, prostate cancer, colorectal cancer, small intestine cancer, lymphatic cancer, ovarian cancer, adenocarcinoma, and asthma; the ovarian cancer includes mucinous ovarian cancer and the peritoneal cancer includes peritoneal pseudomyxoma. The appendix cancer may be, for example, mucinous appendiceal adenocarcinoma, etc.

The serine protease EatA in the present application was disclosed in the study to have a highly efficient and highly specific degradation activity for mucus derived from covered pseudomyxoma, and mucins associated with peritoneal pseudomyxoma are also expressed in large or small amounts associated with disease types in some other mucus diseases. Peritoneal pseudomyxomas that can be treated are mostly pseudomyxomas formed because cancer cells from the colorectum, appendix, and ovary metastasize to the abdominal cavity and cancer cells secrete mucus in large amounts. These cancer cells are diverse in origin and can originate from the colorectal, rectal, pancreatic, breast, lung, gallbladder, and ovary, but a large number of clinical reports have shown that the cancer cells originate from the appendix. These cancer cells secrete large amounts of mucus into the abdominal cavity, which is difficult to degrade, and eventually cause a large amount of accumulation, damage other organs, and increase the severity of disease and mortality. Conventional open surgery to remove mucins often requires multiple procedures due to incomplete treatment and progressive disease, leading to increased morbidity and mortality.

In the above-mentioned definition of mucus-related diseases, mucus in the peritoneal pseudomyxoma refers to a jelly-like semisolid gel material with colorless transparent to amber color formed with MUC2, MUC5AC and MUC5B as the main components formed after a tumor or cancer is formed on the peritoneal surface, which has three phenotypes: soft, medium-hard, and hard. It is characterized by the proliferation of intra-abdominal tumorous mucin secreting cells on the peritoneal surface to produce mucous ascites; mucus of mucinous ovarian cancer refers to the white to amber color formed after the tumor or cancer of ovary is secreted in pathological area, part of the mucus is chocolate-like brown translucent to transparent with extremely high viscosity, and the mucus viscosity is thousands to ten thousand mPa·s syrup-like semi-solid or fluid, characterized by a mass of encapsulated mucin-based viscous mixture attached on the ovarian mucinous cancer cells or glandular sac; the mucus of chronic obstructive pulmonary disease, also known as sputum, is a mucus substance secreted by epithelial/glandular cells in the lung or respiratory tract, characterized by mucin-based mucus adhering to the surface of the respiratory tract and pulmonary airways to form mucus, whereas chronic obstructive pulmonary disease differs from normal physiological conditions of sputum in its higher content and viscosity, often leading to pulmonary and tracheal obstruction.

In general, the administration route of the drug should be carried out based on physical characteristics of the recipient, including the type of disease and the state of health, are considered together, which can be easily concluded by clinical workers and related technical personnel. In some embodiments, the administration route of the drug is any one selected from a group consisting of: injection administration, oral administration, and spray administration.

In the above-mentioned administration routes of the present application, the injection administration may be, for example, intraperitoneal injection administration; the oral administration, for example, as a fast-dissolving tablet, and the spray administration, for example, as an aerosol for nasal or oral inhalation. In particular, for administration by injection, the drug may be carried in a pharmaceutically acceptable carrier or excipient such as physiological saline, phosphate buffer, Tris-HCl buffer, ringer's solution, dextrose injection buffer, and water-propylene glycol solution. Methods for preparing compositions and drugs for parenteral and intraperitoneal administration are readily variable to those skilled in the art.

In the present application, the effective amount of the above-mentioned drugs should be determined depending on various factors such as the type of mucin-related disease to be used and the amount of mucus, and the time expected to be treated. For example, the appropriate amount may depend on a variety of factors including, but not limited to, physical properties of the subject, such as age, weight, and sex; whether the drug is used as a single reagent or as adjuvant therapy; the progression of the disease or condition being treated, i.e. the pathological state; and other factors that will be apparent to those of ordinary skill in the art.

The drug of the present application may be suitable for use in any patient and subject, in some embodiments, the patient is a mammal, most typically a human patient. It will be apparent that other mammalian subjects will benefit from the present application where applicability permits, and therefore, such patients and subjects may be mice, rats, cats, dogs, bears, cattle, horses, and other therapeutically valuable mammals.

The present application has the following beneficial effects: the serine protease described in the present application is the serine protease EatA, which is capable of selectively degrading mucins associated with diseases and has long-term stability, so that the serine protease and the pharmaceutical composition including the serine protease can be used as a drug or a pharmaceutically effective ingredient for treating mucin-secreting cancers such as peritoneal pseudomyxoma (PMP) and other diseases involving mucins.

**BRIEF DESCRIPTION OF THE DRAWINGS**

FIG. 1 shows a result of mucin degradation in a control group of Example 1 without the addition of EatA in the gel-like mucins..

FIG. 2 shows a result of mucin degradation in an experimental group of Example 1 with the addition of EatA in the gel-like mucins.

FIG. 3 shows results of mucin degradation by bromelain (BroM) and serine protease EatA in Example 2.

FIG. 4 shows a temperature curve of mucin degradation by the serine protease EatA in Example 3.

FIG. 5 shows a pH curve of mucin degradation by the serine protease EatA in Example 3.

FIG. 6 is shows a time curve of mucin degradation by the serine protease EatA in Example 3.

FIG. 7 shows results of degradation of miscellaneous proteins mixed inside mucins by bromelain (BroM) and serine protease EatA in Example 4.

FIG. 8 shows results of remaining mucin amounts in the experimental group (mucus + EatA), the control group without enzyme addition (mucus + PBS), and a bromelain experimental group (mucus + bromelain) in Example 5.

FIG. 9 shows results of an intra-abdominal examination of mice in the experimental group (mucus + EatA), the control group without enzyme addition (mucus + PBS), and the bromelain experimental group (mucus + bromelain) in Example 5.

FIG. 10 shows a box plot of a degradation of mucus from ovary mucinous carcinoma and mucinous appendiceal adenocarcinoma by the serine protease EatA in Example 6.

FIG. 11 is a schematic diagram showing results of the degradation of mucus from mucinous appendiceal adenocarcinoma (MAA) by the serine protease EatA in Example 6.

**DETAILED DESCRIPTION**

In order that the present application may be more readily understood, the present application will now be described in further detail with reference to the following examples, which are intended to be illustrative only and are not intended to limit the scope of the present application. Raw materials or components used in the present application can be obtained by commercial routes or conventional methods unless otherwise specified.

Since mucins of different origins and mucins associated with the mucus-related disease have different expression patterns and post-translational modifications, different mucins of clinical pathological origin and mixtures thereof are used in the present application. Preferably, mucins of clinical peritoneal pseudomyxoma origin are used. In the present application, firstly, gel-like mucins from the abdominal cavity of patients with clinical peritoneal pseudomyxoma are collected, after protein precipitation with 30% to 100% ammonium sulfate, cells and tissue fragments, adipose tissue that possibly doped in the collection process of mucins are selected and removed, and then dialyzed and filtered through a 100 KDa dialysis bag. Through the use of sulfate detection test paper, the sulfate concentration in the dialysate and the inner side of the dialysis bag is confirmed. When the sulfate concentration is less than 200 mg/L, the dialysis is terminated and gel-like mucins can be seen. This sample is collected and used as the substrate for in vitro tests to examine the degradation ability of serine protease EatA on mucins. Some examples used mucins removed from the abdominal cavity of clinical patients without purification.

Example 1

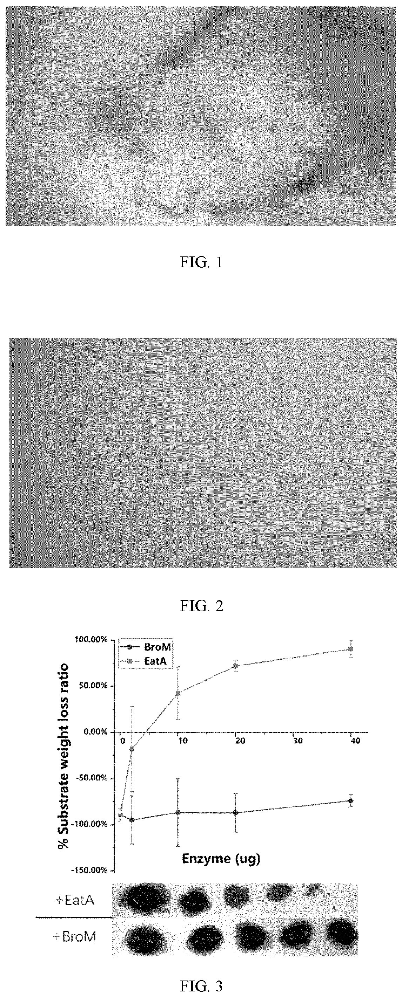

In 500 ul reaction system, 0.30 g of gel-like mucins was accurately weighed and added, about 80 ug of serine protease EatA (reaction coefficient is 133) was added in a experimental group, and serine protease EatA was not added in a control group, then both the experimental group and the control group were incubated at 37°C for 3 h, after the incubation, the degradation of mucins was observed. Results of mucin degradation in the control group and the experimental group were shown in FIG. 1 and FIG. 2, respectively.

It can be seen from FIG. 1 and FIG. 2 that the control group without the addition of serine protease EatA in the gel-like mucins still had a lumpy gel-like protein molecule after 3 h, and could not be pipetted by pipette tip; compared with the control group, the experimental group added with the addition of serine protease EatA in the gel-like mucins showed obvious degradation of gel-like mucins. The gel-like mucins in the experimental group was degraded into a solution similar to water that could be pipetted by the pipette tip, and lump gels disappeared under a stereo microscope.

Example 2

As a broad-spectrum protease, bromelain had extensive degradation activity, which could degrade MUC2 mucin, and had some uses in anti-inflammatory and mucus degradation. Based on this, the example compared the degradation activity of bromelain (BroM) with that of the serine protease EatA on gel-like mucins at the same substrate/enzyme ratio.

0.10 g mucins was accurately weighed and put into a 500 uL phosphate buffer (PBS) reaction system, two enzymes (bromelain (BroM) and serine protease (EatA)) were respectively added to carry out a reaction at 37°C, and the reaction was finished after 6 h. All gel-like products were placed upside down on a 40 um cell strainer, an absorbent paper was used to suck a flowing liquid from the other side of the strainer, the gel-like products were carefully reversed for three to four times, and the flowing liquid was pipetted as much as possible, and the mass of the remaining gel-like mucins was weighed. The above-mentioned test was repeated with three samples at each sampling point, and results were shown in FIG. 3. It can be seen from FIG. 3 that as the mass concentration of EatA enzyme increased within 6 h, the mass loss of gel-like mucins increased, and a residual amount of the gel-like mucins rapidly decreased, namely, the degradation of gel-like mucin; when 40 ug of EatA was added, the mass loss of gel-like mucins was on average 90.33% (calculated based on the initial mass of 0.1 g), and almost all of the gel-like mucins was degraded. In addition, the mass of the gel-like mucins increased at the initial time due to a water absorption of the gel-like mucins. However, bromelain increased the mass of gel-like mucins after absorbing water under this substrate/enzyme ratio. As the BroM enzyme concentration increased, the mass loss of the gel-like mucins was negative, indicating that the degradation efficiency was low and the gel-like mucins were very little degraded.

Example 3

This example characterizes the working environment of the serine protease EatA for the mucin degradation by obtaining a pH-dependent, temperature-dependent, and time-dependent degradation curve of the serine protease EatA for the mucin degradation.

During the experiment, 0.10 g of gel-like mucins was accurately weighed for each single reaction, 20 ug of aminoprotease EatA was added, the reaction was performed in 500 uL of solution system, and the weighing method for the remaining gel-like mucins was the same as that in Example 2. The control group was performed under the same conditions except that the serine protease EatA was not added, and three reactions were set in each group as statistical replicates. Due to the hydrating nature of gel-like mucins, the gel-like mucins absorbed water and swelled when in solution, resulting in a certain weight increase in the control group when finally weighed.

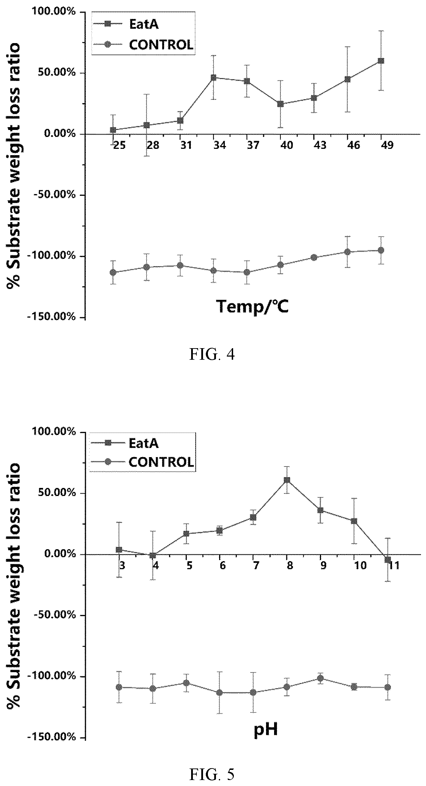

Among them, the experiment on temperature gradient was conducted in normal saline with pH = 7.1, and results were shown in FIG. 4; in the pH experiment, the reaction system used was a solution system with different pH values based on 0.9% normal saline, and the reaction temperature was 37°C, and results are shown in FIG. 5; the reaction time for both the above-mentioned experiments was 6 h.

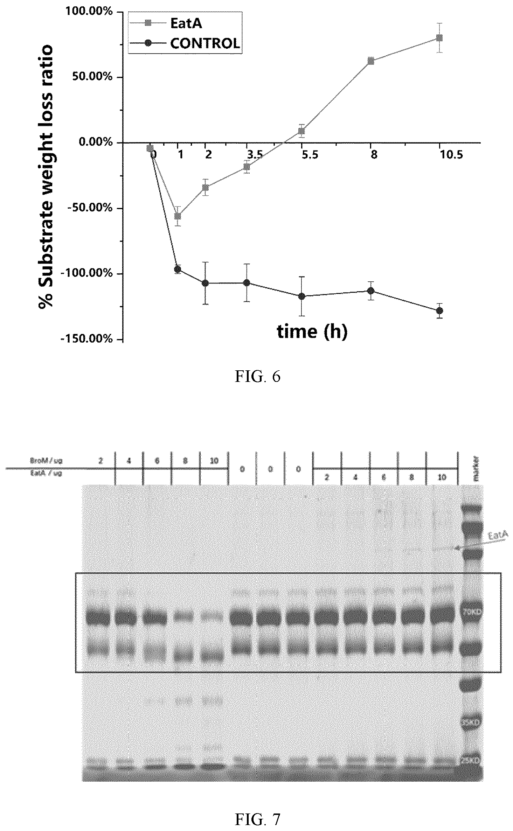

In the experiment regarding the degradation time, based on the temperature curve and pH curve, the experiment was carried out in PBS buffer with pH=7.2-7.4 at 37°C. The results were shown in FIG. 6.

It can be seen from FIG. 4 that with the change of temperature, the amount of gel-like mucins in the control group without the addition of EatA did not change significantly after the end of incubation, while in the experimental group with the addition of EatA, gel-like mucins were degraded and had the maximum enzyme activity at 34-37°C. Since the control group also showed a certain (mass loss) degradation trend above 41°C, which might indicate an effect of this temperature on the protein, it could not be shown that the increased degradation efficiency at this stage was only related to the enzyme.

It can be seen from FIG. 5 that the degradation of gel-like mucins by serine protease EatA under different pH conditions showed that the degradation of gel-like mucins by serine protease EatA had the maximum degradation efficiency around pH = 8. The mass loss of the control group was almost unchanged at different pH.

It can be seen from FIG. 6 that the mass loss of gel-like mucins first decreased to a negative value after the addition of serine protease EatA, indicating that the gel-like mucins absorbed water so that its mass increased, the mass loss increased with the increase of time, and reached 80% at 10.5 h, indicating that the degradation of the gel-like mucins by serine protease EatA continued with the increase of incubation time.

In conclusion, the optimal working environment for serine protease EatA to degrade gel-like mucins is: the reaction temperature is 34-37°C and the pH is 7-8.

Example 4: Specificity of degradation of serine protease EatA

Untreated mucins derived from clinical patients were briefly cleared to remove cellular tissue. 0.20 g of the above-mentioned mucins was accurately weighed and placed in 1 mL of PBS solution, 3-4 magnetic beads with a diameter of 1 mm were added, and then a resulting mixture was frozen and shaken at -30°C for homogenization, so as to destroy the gel-like morphology of mucins, and release the miscellaneous protein from patients with mixed gel-like mucins. 100 uL of the homogenized mucins solution was added with 2-10 ug of protease (bromelain or serine protease EatA), respectively, and the reaction was performed in a 200 uL reaction system. After 6 h of reaction, samples were respectively taken for SDS-PAGE detection, and the total protein after fragmentation, which was the patient-derived miscellaneous protein in the abdominal cavity/abdominal mucins of the patient, was analyzed. The results were shown in FIG. 7.

It can be seen from FIG. 7 that in the group added with BroM (bromelain), at the addition amount of several micrograms of BroM enzyme, the miscellaneous protein derived from the patient's abdominal cavity showed a degradation trend that changed with the increase of the enzyme addition, that is, in the group added with bromelain (BroM), the miscellaneous protein showed degradation. The control group (the band marked "0" in the middle three wells) was consistent with the protein band added with serine protease EatA, indicating that the addition of serine protease EatA did not degrade these proteins derived from abdominal cavity during the degradation of mucins, indicating the specificity of the EatA during the mucin degradation.

Example 5: Ability of serine protease EatA to degrade mucins in vivo

Based on the good results of the in vitro experiments, this example carried out in vivo animal experiments to demonstrate the degradation capacity in vivo of mucins by the serine protease EatA.

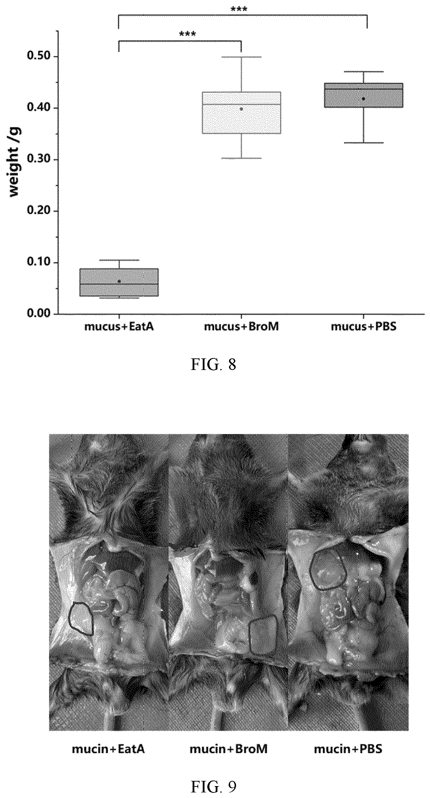

The experiment included twenty C57 male mice aged 6-8 weeks. Five groups were set up in the experiment, including an experimental group (mucus + EatA, 5 mice), a control group (mucus + PBS, 5 mice), a bromelain group (mucus + bromelain, 5 mice), a negative sham operation group (minimal invasive open operation performed without adding mucins and enzyme, 3 mice), and a reference group (minimal invasive open operation performed with adding EatA protease, 2 mice). With reference to the reference average mass of mucins in the abdominal cavity of rat experiment and peritoneal pseudomyxoma patients in a previous study and the reaction coefficient of in vitro experiment, the maximum injection volume in the abdominal cavity in mouse experiment was allowed to be 2 mL, and 0.30 g of human patient-derived gel-like mucins after simple washing with PBS buffer was embedded in the abdominal cavity in mouse experiment, and the living state of the mouse was observed and confirmed. After 24 h, equal amounts of bromelain and serine protease EatA dissolved in PBS, 80 ug, were injected respectively. After 48 h, the mice were sacrificed, laparotomy was performed (results of abdominal examination after laparotomy were shown in FIG. 9) and the whole amount of gel-like mucins in the mice were removed, weighed using the method described in Example 2, counted and compared with the final amount of gel-like mucins remaining in three groups (experimental group (mucus + EatA), control group without enzyme (mucus + PBS) and bromelain group (mucus + bromelain)) to which gel-like mucins was added as a substrate, and results were shown in FIG. 8.

It can be seen from FIG. 8, the group of mice with the addition of the serine protease EatA had the lowest remaining mass of gel-like mucins, indicating that gel-like mucins degraded the most with a degradation efficiency of 78% (calculated according to the initial inoculation amount of 0.30g, gel-like mucins would still absorb water in the abdominal cavity resulting in an increase in its mass). In contrast, the amount of gel-like mucins remaining in the BroM group and the control group was still more than 0.30g, so the degradation efficiency could not be calculated. This indicates that the degradation of gel-like mucins by serine protease EatA was much better than BroM in mice.

The red box in FIG. 9 indicates that the gel-like mucins, after inoculation into the abdominal cavity of mouse, translocates to a different abdominal location as the mouse moves and shows the final volume after degraded, which may correspond to FIG. 8.

Body weight changes of all groups before and after the experiment were recorded during the experiment. There was no difference in the comparison of results. Abdominal examination after sacrificing mice also showed no abnormality. The mice injected with serine protease EatA survived for more than 100 days from the beginning of the experiment without adverse reactions and showed no abnormality in behavior and body weight compared with the non-injected group.

Example 6: Degradation of mucinous ovarian cancer (MOC) mucus, mucoid appendiceal adenocarcinoma (MAA) mucus by serine protease EatA

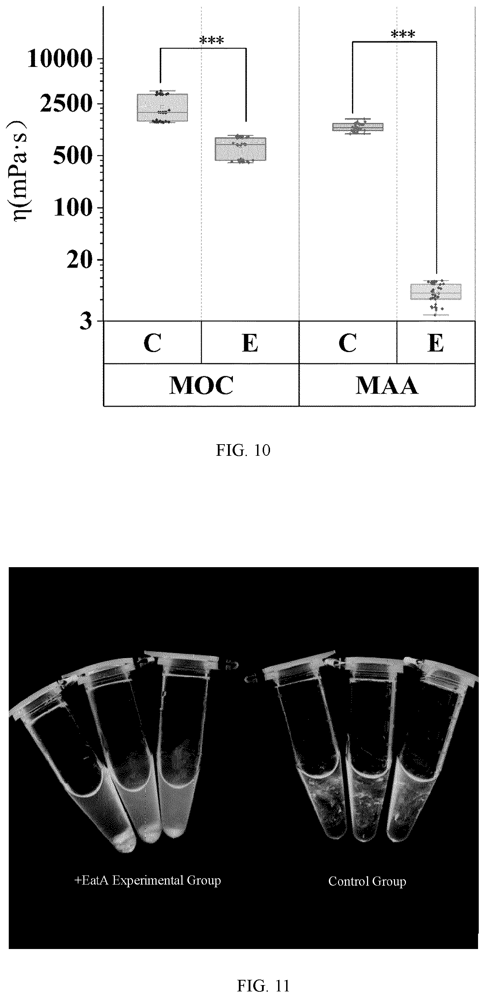

This example carried out a degradation experiment on mucus from mucinous ovarian cancer and mucinous appendiceal adenocarcinoma using the serine protease EatA. 0.50 mL of the above-mentioned two new types of mucinous carcinoma mucus from clinical sources were weighed directly into test tubes, 50 ug of EatA serine protease dissolved in 0.25 mL of PBS buffer was added and then incubated in a total 750 uL reaction system at 37°C, 600 rpm for 20 h. For each type of mucinous carcinoma mucus sample, a control group was set up with only the addition of an equal volume of PBS, for a total of four experiments, each set up with three replicates. The viscosity of the incubated experimental samples was measured under an Anton Paar rheometer, probe model pp25, sample spacing 0.45 mm. After each sample was subjected to pre-shearing at a shearing frequency of 5 rad/s for 20 s, a total of 36 sample point data was obtained for each group of experiments by reading a total of 12 data at a shearing frequency of 10 rad/s every 5 s period for 60 s. The experimental results show the statistical results of all data of each group of samples in a box plot as shown in FIG. 10.

It can be seen from FIG. 10 that in the experiment, the viscosity of mucus of clinically derived mucinous ovarian cancer is on average about 2000 mPa·s, and the viscosity thereof decreases to about 670 mPa·s after incubation with the addition of serine protease EatA; and the viscosity of mucus from mucinous appendiceal adenocarcinoma MAA is about 1200 mPa·s, and the viscosity decreases to about 7 mPa·s after incubation with the addition of serine protease EatA. It can be seen that the viscosity of mucus from the above two different cancer sources is greatly reduced after the reaction, and the viscosity reduction effect of serine protease EatA is extremely obvious.

FIG. 11 further shows a schematic diagram showing the results of the degradation of mucus from mucinous appendiceal adenocarcinoma (MAA). Since the appendix cancer mucus of this sample is transparent, the appearance of reduced viscosity can be detected by examining the homogeneous nature of the solution. It can be seen from the results that after the incubation, the mucus sample solution after the incubation with serine protease EatA becomes uniform, and impurities in the sample which are not degraded by EatA deposit on the bottom after standing. While the control group also maintained a high degree of gel-like mucus, and internal impurities in the sample could not be deposited on the bottom after standing.

At the same time, the serine protease EatA in the present application is proven to have a certain degradation effect on mucus (sputum), mucous colon cancer and the like of patients with chronic obstructive pulmonary pneumonia in the relevant study results of the applicant. According to the above examples, the serine protease of the present application has a good medical utilization prospect and has great advantages compared with other drugs and preparations to be formulated currently on the market.

It should be noted that the above-mentioned embodiments are merely illustrative of the present application and do not constitute any limitation of the present application. The present application has been described with reference to exemplary embodiments, but it is to be understood that the words which have been used are words of description and illustration, rather than words of limitation. It is intended that the present application be construed as including all such modifications and alterations insofar as they come within the scope of the appended claims or the equivalents thereof. Although the present application is described herein with reference to particular methods, materials, and embodiments, the present application is not intended to be limited to the particular disclosed examples, on the contrary, the present application extends to all other methods and uses having the same function.

## Claims

1. A serine protease selectively degrading mucins, **characterized in that** the serine protease is serine protease EatA, a protein of the serine protease EatA comprises a passenger domain with serine protease EatA activity, and an amino acid sequence of the passenger domain is an amino acid sequence with at least 90% identity to SEQ ID NO 2.

2. The serine protease according to claim 1, **characterized in that** the amino acid sequence of the passenger domain is an amino acid sequence as shown in SEQ ID NO 2.

3. The serine protease according to claim 1, **characterized in that** the protein of the serine protease EatA further comprises at least one of a signal peptide at an N-terminal or a beta barrel structure at a C-terminal.

4. The serine protease according to claim 3, **characterized in that** an amino acid sequence of the serine protease EatA having the passenger domain and the beta barrel structure at the C-terminal is an amino acid sequence with at least 90% identity to SEQ ID NO 3.

5. The serine protease according to claim 3, **characterized in that** an amino acid sequence of the serine protease EatA having the passenger domain and the beta barrel structure at the C-terminal is an amino acid sequence as shown in SEQ ID NO 3.

6. The serine protease according to claim 3, **characterized in that** a full-length amino acid sequence of the serine protease EatA having the passenger domain, the signal peptide at the N-terminal, and the beta barrel structure at the C-terminal is an amino acid sequence with at least 80% identity to SEQ ID NO. 1.

7. The serine protease according to claim 3, **characterized in that** a full-length amino acid sequence of the serine protease EatA having the passenger domain, the signal peptide at the N-terminal, and the beta barrel structure at the C-terminal is an amino acid sequence with at least 90% identity to SEQ ID NO 1.

8. The serine protease according to claim 3, **characterized in that** a full-length amino acid sequence of the serine protease EatA having the passenger domain, the signal peptide at the N-terminal, and the beta barrel structure at the C-terminal is an amino acid sequence as shown in SEQ ID NO 1.

9.  The serine protease according to claim 1, **characterized in that** the serine protease EatA has a specific serine protease activity for selectively degrading mucins; the mucins that is degraded is at least one of mucins present in peritoneal pseudomyxoma mucus, chronic obstructive pulmonary disease mucus, mucinous ovarian cancer mucus, mucinous colorectal cancer mucus, or appendix cancer mucus.

10. A pharmaceutical composition, **characterized by** comprising the serine protease according to claim 1 and an additional reagent, and the additional reagent is at least one selected from a group consisting of: a chemotherapeutic and radiotherapeutic reagent, an enzyme, and a chemical salt reagent; wherein the chemotherapeutic and radiotherapeutic reagent is at least one selected from a group consisting of: gemcitabine, cisplatin, adriamycin, fluorouracil, taxodone, paclitaxel, and oxaliplatin; the enzyme is at least one selected from a group consisting of: N-acetylgalactosidase, galactosidase, glucosidase, sialidase and specific endomucinase; and the chemical salt reagent is at least one selected from a group consisting of: sodium bicarbonate, carbocysteine, N-acetylcysteine and ambroxol.

11. Use of the serine protease according to claim 1 or the pharmaceutical composition according to claim 10 in preparing a drug for at least one of treating mucus-related diseases, or improving a therapeutic effect of the mucus-related diseases and a quality of life of a patient with the mucus-related diseases; wherein the mucus-related disease is at least one selected from a group consisting of: chronic obstructive pulmonary disease, mucinous colorectal cancer, lung cancer, liver cancer, gastric cancer, appendix cancer, peritoneal cancer, prostate cancer, colorectal cancer, small intestine cancer, lymphatic cancer, ovarian cancer, adenocarcinoma, and asthma; the ovarian cancer comprises mucinous ovarian cancer and the peritoneal cancer comprises peritoneal pseudomyxoma.

12. The use according to claim 11, **characterized in that** an administration route of the drug is any one selected from a group consisting of: injection administration, oral administration, and spray administration; preferably, the injection administration is intraperitoneal injection administration.

FIG. 1

FIG. 2

FIG. 3

FIG. 4

FIG. 5

FIG. 6

FIG. 7

FIG. 8

FIG. 9

FIG. 10

FIG. 11

## INTERNATIONAL SEARCH REPORT

| | International application No. |
|---|---|
| | **PCT/CN2023/129169** |

**A. CLASSIFICATION OF SUBJECT MATTER**

C12N 9/52(2006.01)i; A61K 38/48(2006.01)i; A61P 35/00(2006.01)i; A61P 11/06(2006.01)i

According to International Patent Classification (IPC) or to both national classification and IPC

**B. FIELDS SEARCHED**

Minimum documentation searched (classification system followed by classification symbols)

IPC: C12N A61K A61P

Documentation searched other than minimum documentation to the extent that such documents are included in the fields searched

Electronic data base consulted during the international search (name of data base and, where practicable, search terms used)

CNTXT; VCN; VEN; ENTXT; ENTXTC; OETXT; CNKI; 万方, WANFANG; 读秀学术, DUXIU SCHOLAR; 中国专利生物序列检索系统, China Patents Biological Sequence Search System; NCBI; STNEXT; ISI Web of Science; Springer: 孙正龙, 刘俊, 张聪, 陈凯, 丝氨酸蛋白酶, EatA, 乘客域, 粘液, 粘蛋白, 降解, 癌症, Sun Zhenglong, Liu Jun, Zhang Cong, Chen Kai, serine protease, passenger domain, mucus, mucin, degrade, degradtion, cancer, 基于序列1-3的检索, search on the basis of sequences 1-3

**C. DOCUMENTS CONSIDERED TO BE RELEVANT**

| Category* | Citation of document, with indication, where appropriate, of the relevant passages | Relevant to claim No. |
|---|---|---|
| PX | CN 115786311 A (SUN ZHENGLONG) 14 March 2023 (2023-03-14) <br> claims 1-10 | 1-12 |
| X | Seema K. Patel et al. "Identification and Molecular Characterization of EatA, an Autotransporter Protein of Enterotoxigenic Escherichia coli" <br> *INFECTION AND IMMUNITY,* Vol. 72, No. 3, 31 March 2004 (2004-03-31), pages 1786-1794 <br> page 1789, "Result" section, page 1790, and figure 2 | 1-9 |
| Y | Seema K. Patel et al. "Identification and Molecular Characterization of EatA, an Autotransporter Protein of Enterotoxigenic Escherichia coli" <br> *INFECTION AND IMMUNITY,* Vol. 72, No. 3, 31 March 2004 (2004-03-31), pages 1786-1794 <br> page 1789, "Result" section, page 1790, and figure 2 | 10-12 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 December 2023** | **13 December 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/129169** |

### C. DOCUMENTS CONSIDERED TO BE RELEVANT

| Category* | Citation of document, with indication, where appropriate, of the relevant passages | Relevant to claim No. |
| --- | --- | --- |
| Y | Pardeep Kumar et al. "EatA, an Immunogenic Protective Antigen of Enterotoxigenic Escherichia coli, Degrades Intestinal Mucin" *Infection and Immunity,* Vol. 82, No. 2, 30 November 2013 (2013-11-30), pages 500-508 page 500, abstract, page 501, and figure 1 | 10-12 |
| A | CN 112236171 A (MUCPHARM PTY LTD.) 15 January 2021 (2021-01-15) claims 1-34 | 1-12 |
| A | WO 2016115328 A1 (UNIVERSITY OF WASHINGTON) 21 July 2016 (2016-07-21) claims 1 and 5-10 | 1-12 |

Form PCT/ISA/210 (second sheet) (July 2022)

## INTERNATIONAL SEARCH REPORT

| International application No. |
| --- |
| **PCT/CN2023/129169** |

**Box No. I**       **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    ☑ forming part of the international application as filed.

     b.    ☐ furnished subsequent to the international filing date for the purposes of international search (Rule 13*ter*.1(a)),

          ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2023/129169**

| Patent document cited in search report | | | Publication date (day/month/year) | Patent family member(s) | | | Publication date (day/month/year) |
|---|---|---|---|---|---|---|---|
| CN | 115786311 | A | 14 March 2023 | None | | | |
| CN | 112236171 | A | 15 January 2021 | AU | 2019225453 | A1 | 08 October 2020 |
| | | | | JP | 2021515042 | A | 17 June 2021 |
| | | | | SG | 11202007976 | WA | 29 September 2020 |
| | | | | EP | 3755376 | A1 | 30 December 2020 |
| | | | | EP | 3755376 | A4 | 01 December 2021 |
| | | | | WO | 2019161457 | A1 | 29 August 2019 |
| | | | | US | 2021008180 | A1 | 14 January 2021 |
| WO | 2016115328 | A1 | 21 July 2016 | US | 2018000918 | A1 | 04 January 2018 |

Form PCT/ISA/210 (patent family annex) (July 2022)